# EUROPÄISCHE PATENTSCHRIFT

(11) **EP 3 157 900 B1**
(45) Veröffentlichungstag und Bekanntmachung des Hinweises auf die Patenterteilung: **15.05.2019**
(21) Anmeldenummer: 15728903.4
(22) Anmeldetag: 17.06.2015
(51) Int. Cl.: C07C 67/307, C07C 67/32, C07C 69/63

(54) **VERFAHREN ZUR HERSTELLUNG VON 2-HALOGENACRYLSÄUREESTERN**
PROCESS FOR THE PREPARATION OF 2-HALO ACRYLIC ACID ESTERS
PROCÉDÉ DE PRÉPARATION D'ESTER D'ACID 2-HALOGÈNE ACRYLIQUE

(30) Priorität: 18.06.2014 EP 14172874
(43) Veröffentlichungstag der Anmeldung: 26.04.2017
(73) Patentinhaber: Saltigo GmbH, 51369 Leverkusen (DE)
(72) Erfinder: VON DEM BRUCH, Karsten, 51371 Leverkusen (DE); SCHERWITZKI, Andreas, 42897 Remscheid (DE)
(74) Vertreter: Matzke, Michael
(86) Internationale Anmeldenummer: PCT/EP2015/063634
(87) Internationale Veröffentlichungsnummer: WO 2015/193392

(56) Entgegenhaltungen:
- EP-A2- 0 203 462
- EP-A2- 0 249 867
- GB-A- 1 115 287
- DATABASE REAXYS [Online] Elsevier Information Systems GmbH, Frankfurt/Main (DE); XP002728398, Database accession no. 7400708, 7141158 (Rx-IDs) -& GAULT, BOUVIER: "Nouvellle synthèse des esters alpha-fluoracryliques et de leurs dérivés", COMPTES RENDUS DES SEANCES DE L'ACADEMIE DES SCIENCES, SERIE C: SCIENCES CHIMIQUES, Bd. 70, Nr. 105896v, 27. Januar 1969 (1969-01-27), Seiten 354-357, XP009179642,
- SURYA PRAKASH ET AL.: "Direct Electrophilic Monofluoromethylation", ORG. LETT., Bd. 10, Nr. 4, 21. Februar 2008 (2008-02-21), Seiten 557-560, XP002660281, ISSN: 1523-7060, DOI: 10.1021/OL702500U [gefunden am 2008-01-17]
- DATABASE REAXYS [Online] Elsevier Information Systems GmbH, Frankfurt/Main (DE); XP002728399, Database accession no. 1785448, 4977083, 5000762 (RIDs) & PROSYANIK ET AL.: ZHURNAL ORGANICHESKOI KHIMII, Bd. 19, Nr. 12, 1983, Seiten 2480-2484,
- DATABASE REAXYS [Online] Elsevier Information Systems GmbH, Frankfurt/Main (DE); XP002728400, Database accession no. 22451468, 932651, 1255742 (Rx-IDs) & YAKUBOVICH, BELYAEVA: J. GEN. CHEM. USSR (ENGL. TRANSL.), Bd. 31, 1961, Seiten 2119-2122,
- KRAPCHO: "SYNTHETIC APPLICATIONS OF DEALKOXYCARBONYLATIONS OF MALONATE ESTERS, BETA-KETO ESTERS, ALPHA-CYANO ESTERS AND RELATED COMPOUNDS IN DIPOLAR APROTIC MEDIA - PART I", SYNTHESIS, 1. Oktober 1982 (1982-10-01), Seiten 805-822, XP000999104, ISSN: 0039-7881, DOI: 10.1055/S-1982-29953
- KRAPCHO: "Recent synthetic applications of the dealkoxycarbonylation reaction. Part 1. Dealkoxycarbonylations of malonate esters", ARKIVOC, Bd. 2007, Nr. Part (ii): Special Issue 'Reviews and Account, 1. Januar 2007 (2007-01-01), Seiten 1-53, XP055134568, ISSN: 1551-7004
- MCMURRY: "ESTER CLEAVAGES VIA SN2-TYPE DEALKYLATION" In: Organic Reactions: 1. Januar 1976 (1976-01-01), XP000999838, Seiten 187-224, The whole document. Cited as common general knowledge.
- SALOMON ET AL.: "Recent developments in chemical deprotection of ester functional groups", TETRAHEDRON, Bd. 49, Nr. 18, 30. April 1993 (1993-04-30), Seiten 3691-3734, XP026596583, ISSN: 0040-4020, DOI: 10.1016/S0040-4020(01)90225-X [gefunden am 1993-04-30]

## Beschreibung

Die vorliegende Erfindung betrifft ein Verfahren zur Herstellung von 2-Halogenacrylsäureestern aus 2-Hydroxymethyl- oder 2-Halogenmethyl- oder 2-Chlorsulfinyloxymethyl-2-halogenmalonsäurediestern. Ein weiterer Gegenstand der Erfindung sind neuartige 2-Halogenmethyl-2-halogen-malonsäurediester oder 2-Chlorsulfinyloxymethyl-2-halogenmalonsäurediester, die zur Herstellung der 2-Halogenacrylsäureester eingesetzt werden können.

Substituierte 2-Halogenacrylsäureester, insbesondere 2-Fluoracrylsäureester stellen Edukte zur Synthese von Polymeren dar. Diese können z.B. als Kunststoffe in optischen Lichtwellenleitern und als polymere Zusatzstoffe in Arzneimitteln eingesetzt werden.

Aus der Literatur sind verschiedene Verfahren zur Herstellung von 2-Fluoracrylsäureestern bekannt.

Aus Journal of Fluorine Chemistry, 55, 1991, S. 149 - 162 ist ein Verfahren zur Herstellung von 2-Fluor-acrylsäureestern durch Hydrolyse von α-Hydroxymethyl-α-fluormalonsäureestern, anschließender Decarboxylierung und erneuter Veresterung bekannt. Das Verfahren weist den Nachteil auf, dass lediglich geringe Ausbeuten erhalten werden.

Weiterhin ist aus JP 2001172223 bekannt substituierte 2-Fluoracrylsäureester aus 2,2-Brom-fluorpropionsäureestern herzustellen. Nachteilig an diesem Verfahren ist, dass die Edukte kaum verfügbar sind und nur geringe Ausbeuten erhalten werden, das Verfahren folglich unökonomisch ist.

In EP 415 214 A wird ein vierstufiges Verfahren zur Herstellung eines 2-Fluoracrylsäureesters ausgehend von 2,3-Dichlor-1-propen beschrieben. Weitere Verfahren zur Herstellung von 2-Fluoracrylsäure-Derivaten ausgehend von 3-Hydroxy-2-fluorpropionaten durch Umsetzung mit Toluolsulfonsäurechlorid und Eliminierung des entstandenen Tosylates in Gegenwart von Kaliumphthalimid sind aus Journal of Fluorine Chemistry, 1993, 60, S. 149-162 und aus Coll. Czech. Chem. Commun., 1983, 48, S. 319-326 bekannt. Vorgenannte Verfahren haben gemeinsam, dass sie aus ökonomischen und sicherheitstechnischen Gründen in technischen Prozessen nicht wünschenswert sind.

Ein weiteres Verfahren zur Herstellung von 2-Fluor-acrylsäure-Derivaten durch Umsetzung von 3-Hydroxy-2-fluorpropionaten mit Wasser-entziehenden Mitteln ist aus Bull. Soc. Chem. Fr., 1975, S. 1633 - 1638 bekannt. Nachteilig an diesem Verfahren ist ebenfalls die geringe Produktausbeute.

Aus EP 249 867 A und EP 203 462 A sind Verfahren zur Herstellung von 2-Fluor-acrylsäureestern bekannt bei dem man 2-Fluormalonsäuredimethylester in einem ersten Verfahrensschritt mit Formaldehyd zu 2-Hydroxymethyl-2-fluor-malonsäuredimethylester umsetzt, der in einem zweiten Schritt unter Decarboxylierung und Dehydratisierung in 2-Fluoracrylsäure überführt wird. In einen dritten Schritt werden durch Veresterung der 2-Fluoracrylsäure mit einem Alkohol die entsprechenden 2-Fluoracrylsäureester erhalten.

Auch diesen Verfahren ist gemeinsam, dass sie entweder aus sicherheitstechnischen Gründen für eine Herstellung von 2-Halogenacrylsäureestern ungeeignet sind oder aufgrund zu vieler Verfahrensstufen zu geringe Reaktionsausbeuten liefern.

Es bestand daher weiterhin ein Bedürfnis nach einem Verfahren zur Herstellung von 2-Halogen-acrylsäureestern, das die Nachteile des Standes der Technik überwindet und mit dem substituierte 2-Halogenacrylsäureester in effizienter Weise in technisch gangbaren Prozessen hergestellt werden können.

Gegenstand der Erfindung ist daher ein Verfahren zur Herstellung von 2-Halogenacrylsäureestern der Formel (IV) 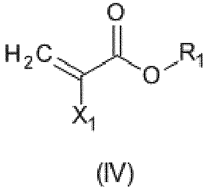 worin
- R₁: für C₁-C₆-Alkyl, oder C₃-C₆-Cycloalkyl und
- X₁: für Fluor steht,
umfassend den Schritt
a) Umsetzung von Verbindungen der Formel (II), 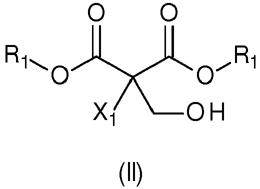 und /oder Verbindungen der Formel (III), 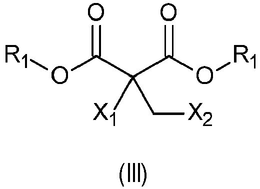
   in denen jeweils R₁ und X₁ die für Formel (IV) genannte Bedeutung besitzen und die beiden Reste R₁ identisch oder verschieden, vorzugsweise identisch sind,
   und wobei X₂ in Formel (III) für Chlor, Chlorsulfinyloxy oder Brom steht,
   in Gegenwart von Base ausgewählt aus der Gruppe bestehend aus Erdalkali- oder Alkalimetallhydroxide, -amide, -alkoholate, -carbonate, -hydrogenphosphate, -phosphate und in Gegenwart von Lösungsmitteln ausgewählt aus der Gruppe bestehend aus Ethern, Amiden, Sulfonen und Sulfoxiden zu Verbindungen der Formel (IV) bei Temperaturen von 120°C bis 170°C.

In Gegenwart der erfindungsgemäßen Base werden die Verbindungen der Formel (IV) aus den Verbindungen der Formel (II) unter Kohlenstoffdioxid- und Alkoholabspaltung (R₁OH) erhalten.

In Gegenwart der erfindungsgemäßen Base werden die Verbindungen der Formel (IV) aus den Verbindungen der Formel (III) unter Kohlenstoffdioxid- und Methylhalogenidabspaltung (CH₃X₂) erhalten.

In einer weiteren Ausführungsform werden die Verbindungen der Formel (IV) aus den Verbindungen der Formel (III), wenn X₂=Chlorsulfinyloxy ist, unter Kohlenstoffdioxid-, Schwefeldioxid und Methylchloridabspaltung erhalten. Unter Chlorsulfinyloxy wird der Rest -O-SO-Cl verstanden.

Bevorzugt steht R₁ für Methyl, Ethyl, n-Propyl, i-Propyl, n-, i-, s- oder t-Butyl, n-Pentyl oder n-Hexyl, insbesondere für Methyl oder Ethyl. Besonders bevorzugt steht R₁ für Methyl.

In einer Ausführungsform steht X₂ in den angegebenen Formeln (I) bis (IV) für Chlor oder Brom. Besonders bevorzugt steht X₂ für Chlor. In einer weiteren Ausführungsform steht X₂ in den angegebenen Formeln (I) bis (IV) für Fluor, Chlor, Chlorsulfinyloxy oder Brom.

Besonders bevorzugte Verbindungen der Formel (IV) sind 2-Fluoracrylsäuremethylester und 2-Fluoracrylsäureethylester, wobei 2-Fluoracrylsäuremethylester weiter bevorzugt ist.

Besonders bevorzugte Verbindungen der Formel (II) sind 2-Fluor-2-hydroxymethyl-malonsäuredimethylester und 2-Fluor-2-hydroxymethyl-malonsäurediethylester, wobei 2-Fluor-2-hydroxymethyl-malonsäuredimethylester weiter bevorzugt ist.

Besonders bevorzugte Verbindungen der Formel (III) sind 2-Fluor-2-chlormethyl-malonsäuredimethylester und 2-Fluor-2-chlormethyl-malonsäurediethylester, wobei 2-Fluor-2-chlormethyl-malonsäuredimethylester weiter bevorzugt ist. Darüber hinaus besonders bevorzugte Verbindungen der Formel (III) sind 2-Fluor-2-chlorsulfinyloxy-methylmalonsäurediethylester und 2-Fluor-2-chlorsulfinyloxymethyl-malonsäure-dimethylester.

Die Herstellung der als Edukt eingesetzten Verbindungen der Formel (II) ist durch Hydroxymethylierung von 2-Halogen-malonsäuredialkylestern der Formel (I) mit Formaldehyd aus EP 249 867 A und EP 203 462 A allgemein bekannt. Die Verbindungen der Formel (I) 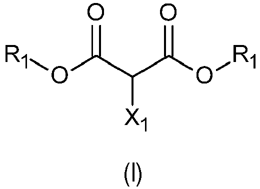 in der R₁ und X₁ die oben angegebenen Bedeutungen haben, sind ebenfalls bekannt und können nach bekannten Verfahren hergestellt werden (z.B. aus den entsprechenden Malonsäuredialkylestern durch Halogenierung oder aus 2-Chlor-malonsäuredialkylestern durch Halogen-Austausch).

Die Herstellung der als Edukt eingesetzten Verbindungen der Formel (III) kann durch Umsetzung von Verbindungen der Formel (II) mit einem Halogenierungsmittel erfolgen.

Als Halogenierungsmittel können Fluorierungs-, Chlorierungs- oder Bromierungsmittel eingesetzt werden. Halogenierungsmittel umfassen beispielsweise Thionylchlorid, Thionylbromid, Phosphortrichlorid, Phosphortribromid, Sulfurylchlorid, Sulfurylbromid und Halogenwasserstoffe wie Fluor-, Chlor- oder Bromwasserstoff. Ein besonders bevorzugtes Halogenierungs- bzw. Chlorierungsmittel ist Thionylchlorid.

Das Halogenierungsverfahren kann in Gegenwart oder in Abwesenheit eines Lösungsmittels durchgeführt werden.

Geeignete Lösungsmittel für die Halogenierung umfassen beispielsweise Chlorkohlenwasserstoffe wie beispielsweise Methylenchlorid, Chloroform, Tetrachlorkohlenstoff; aromatische, gegebenenfalls chlorierte Kohlenwasserstoffe wie beispielsweise Toluol, Xylol, Chlorbenzol, 1,2-Dichlorbenzol, 1,3-Dichlorbenzol und 1,4-Dichlorbenzol; aliphatische Kohlenwasserstoffe wie beispielsweise Hexan, Heptan, Cyclohexan und Methylcyclohexan oder Mischungen der vorgenannten Lösungsmittel.

Das Halogenierungsverfahren kann in Gegenwart oder in Abwesenheit einer Base erfolgen. Geeignete Basen umfassen beispielsweise Natrium- oder Kaliumhydroxid, Natrium- oder Kaliumcarbonat, Natrium- oder Kaliumalkoxid, Ammoniak, organische Amine wie Triethylamin, Tributylamin oder Pyridin, oder Mischungen der vorgenannten Basen.

Das Halogenierungsverfahren kann beispielsweise bei einer Temperatur im Bereich von -20°C bis 200°C, vorzugsweise bei 50°C bis 90°C, oder darüber hinaus vorzugsweise bei 0°C bis 50°C, oder weiterhin darüber hinaus vorzugsweise bei 80°C bis 120°C, oder weiterhin darüber hinaus vorzugsweise bei 110°C bis 170°C durchgeführt werden.

Bei der Umsetzung der Verbindung der allgemeinen Formel (II) mit Thionylchlorid kann als Produkt ein 2-Fluor-2-chlorsulfinyloxymethyl-malonsäurediester erhalten werden (X₂= O-SO-Cl). Diese Verbindung wird beispielsweise bei der Umsetzung von der Verbindung der allgemeinen Formel (II) mit Thionylchlorid bei Temperaturen von 0 bis 50°C erhalten. Bei der Umsetzung der Verbindung der allgemeinen Formel (II) mit Thionylchlorid in Anwesenheit einer Base bei höheren Temperaturen, beispielsweise bei Temperaturen von 80 bis 120°C, wird dagegen der entsprechende Chlormethylmalonsäurediester erhalten. In Abwesenheit einer Base wird bei der Umsetzung der Verbindung der allgemeinen Formel (II) mit Thionylchlorid dagegen der entsprechende Chlormethylmalonsäurediester erst bei höheren Temperaturen, beispielsweise bei Temperaturen von 110 bis 170°C erhalten.

Die alternativ oder zusätzlich als Edukt in das erfindungsgemäße Verfahren eingesetzten Verbindungen der Formel (III) sind neu und daher ebenso wie ihre Herstellung von der Erfindung mit umfasst.

In einer bevorzugten Ausführungsform werden als Edukt in das erfindungsgemäße Verfahren die Verbindungen der Formel (III) eingesetzt.

Der Vorteil zum bekannten Stand der Technik ist darin zu sehen, dass das erfindungsgemäße Verfahren nicht über die Herstellung der freien 2-Halogenacrylsäure verläuft. Ein zusätzlicher Veresterungsschritt entfällt somit.

Bevorzugt werden als Basen Alkalimetallhydroxide ausgewählt aus Natriumhydroxid oder Kaliumhydroxid, Alkalimetallamide ausgewählt aus Natriumamid oder Lithiumdiethylamid, Alkalimetallalkoholate ausgewählt aus Natriummethylat oder Kalium-tert-butylat, Alkalimetall-carbonate ausgewählt aus Natriumcarbonat oder Kaliumcarbonat, Alkalimetallhydrogenphosphate ausgewählt aus Dinatriumhydrogenphosphat, Dikaliumhydrogenphosphat, Natriumdihydrogenphosphat oder Kaliumdihydrogenphosphat, oder Alkalimetallphosphate ausgewählt aus Trinatriumphosphat, Trikaliumphosphat eingesetzt.

Besonders bevorzugt werden als Basen Natrium- oder Kaliumhydrogencarbonat eingesetzt.

Das Verfahren wird erfindungsgemäß in Gegenwart von Lösungsmittel ausgewählt aus der Gruppe bestehend aus Ethern, Amiden, Sulfonen und Sulfoxiden durchgeführt.

Geeignete Lösungsmittel umfassen aus der Gruppe der Ether beispielsweise Dioxan, aus der Gruppe Amide beispielsweise N,N-Dimethyl-formamid, N,N Dimethyl-acetamid und N-Methyl-pyrrolidon, aus der Gruppe Sulfone beispielsweise Sulfolan; und aus der Gruppe Sulfoxide beispielsweise Dimethylsulfoxid. Ein besonders bevorzugtes Lösungsmittel ist N-Methyl-pyrrolidon.

Das Verfahren wird erfindungsgemäß bei einer Temperatur im Bereich von 120 bis 170°C, vorzugsweise bei 140 bis 160°C, durchgeführt.

Mit Hilfe des erfindungsgemäßen Verfahrens ist die Herstellung von 2-Halogenacrylsäureestern der Formel (IV) in vorteilhafter Weise weitgehend ohne die Bildung störender Nebenprodukte möglich.

Die Aufreinigung der 2-Halogenacrylsäureestern der Formel (IV) kann nach dem Fachmann bekannten Verfahren, beispielsweise durch Extraktion mit Lösungsmitteln oder bevorzugt durch Destillation erfolgen. Die Weiterverarbeitung der 2-Halogenacrylsäureestern der Formel (IV) aus dem erfindungsgemäßen Verfahren kann aber auch ohne weitere Aufarbeitung erfolgen.

Durch das erfindungsgemäße Verfahren können 2-Halogenacrylsäureestern der Formel (IV) in hohen Ausbeuten und in hoher Reinheit auf technisch einfache und sichere Weise hergestellt werden. Das erfindungsgemäße Verfahren erfordert nicht die Handhabung von Chemikalien, die wegen ihres Gefahrenpotenzials besonderen Aufwand erfordern und ist ohne Probleme auch in größerem Maßstab durchführbar. Es ist vor allem überraschend, dass das erfindungsgemäße Verfahren 2-Halogenacrylsäureestern der Formel (IV) in hohen Ausbeuten und in hoher Reinheit liefert.

Die erfindungsgemäß hergestellten 2-Halogenacrylsäureester der Formel (IV) eignen sich insbesondere zur Herstellung von Kunststoffen und polymeren Zusatzstoffen in Arzneimitteln.

Ein weitere Gegenstand der Erfindung sind 2-Chlorsulfinyloxymethyl-2-halogenmalonsäuredialkylesterder Formel (III) 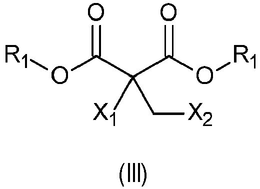 in der
- R₁: für C₁-C₆-Alkyl, oder C₃-C₆-Cycloalkyl und
- X₁: für Fluor steht, und
- X₂: für Chlorsulfinyloxy steht.

Bevorzugt ist R₁ eine Ethyl-oder Methylgruppe, besonders bevorzugt eine Methylgruppe. 2-Chlorsulfinyloxymethyl-2-halogen-malonsäuredialkylesterder der Formel (III) waren bisher nach dem Stand der Technik nicht bekannt. Wie vorstehend beschrieben, können sie als Ausgangsprodukte für die Herstellung von 2-Halogenacrylsäureestern eingesetzt werden.

Ein weiterer Gegenstand der Erfindung ist ein Verfahren zur Herstellung von 2-Chlorsulfinyloxymethyl-2-halogen-malonsäuredialkylestern der Formel (III) 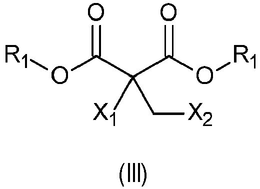 (X₂ = Chlorsulfinyloxy) durch Umsetzung von 2-Hydroxymethyl-2-halogen-malonsäuredialkylestern der Formel (II) 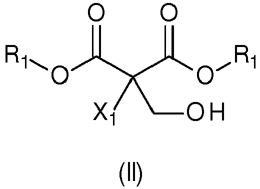 mit Thionylchlorid bei Temperaturen von 0 bis 50°C, wobei in den Formeln (II) und (III) R₁, X₁ und X₂ die oben angegebene Bedeutung haben.

Bevorzugt ist R₁ eine Ethyl-oder Methylgruppe, besonders bevorzugt eine Methylgruppe.

Die nachstehenden Beispiele dienen der Erläuterung der Erfindung, ohne sie dabei zu beschränken.

### Beispiele

### Beispiel 1: Herstellung von 2-Fluor-2-hydroxymethyl-malonsäuredimethylester (Analog wie in EP-A-0203462 oder EP-A-0249867 beschrieben).

Zu einer bei Raumtemperatur hergestellten Lösung von 8 g Kaliumhydrogencarbonat in 80 g einer 30%-igen wässrigen Formaldehyd-Lösung wurden bei einer Innentemperatur von 20 bis 25°C 100 g 2-Fluormalonsäuredimethylester innerhalb von 1 Stunde zudosiert. Nach 1 Stunde Nachrühren bei Raumtemperatur wurden 112 g Produkt in einer Reinheit von ca. 88 % durch Extraktion mit Ethylacetat und Eindampfen des Extrakts in Form eines farblosen Öls gewonnen, das bei Raumtemperatur rasch zu einem farblosen Feststoff erstarrte. Das Produkt wurde zur weiteren Aufreinigung aus Toluol umkristallisiert.

### Beispiel 2: Herstellung von 2-Fluor-2-hydroxymethyl-malonsäurediethylester (Analog wie in EP-A-0203462 oder EP-A-0249867 beschrieben).

Zu einer bei Raumtemperatur hergestellten Lösung von 7 g Kaliumhydrogencarbonat in 70 g einer 30%-igen wässrigen Formaldehyd-Lösung wurden nach Zusatz von 7,0 g Ethanol 100 g 2-Fluormalonsäurediethylester innerhalb von 1 Stunde bei einer Innentemperatur von 20 bis 25°C zudosiert. Nach 3 Stunden Nachrühren bei Raumtemperatur wurden 122 g Produkt in einer Reinheit von ca. 84 % durch Extraktion mit Ethylacetat und Eindampfen des Extrakts in Form eines blass beigen Öls gewonnen. Das Produkt wurde zur weiteren Aufreinigung aus Toluol umkristallisiert.

### Beispiel 3: Herstellung von 2-Fluor-2-chlormethyl-malonsäuredimethylester (erfindungsgemäß)

60 g des Produkts aus Beispiel 1 wurden bei 0 bis 5°C mit 300 g Thionylchlorid versetzt, wobei die primär auftretende Suspension nach kurzer Zeit in eine klare, hell-beige Lösung überging. Nach 1 Stunde Nachrühren bei 0 bis 5°C wurden bei dieser Temperatur 1,8 g Triethylamin zugesetzt, die Reaktionsmischung zum Rückfluss aufgeheizt und für 48 Stunden unter Rückfluss erhitzt. Der nach anschließender Einengung bis zur Trockne verbliebene Rückstand wurde in 200 g Dichlormethan aufgenommen und die resultierende Lösung bei Raumtemperatur mit 200 g einer 5 Gew.-%-igen wässrigen Natriumhydrogencarbonat-Lösung neutral gewaschen. Die organische Phase wurde erneut zur Trockne eingeengt und der verbliebende Rückstand bei ca. 10 mbar fraktioniert destilliert. Man erhielt knapp 50 g eines gelblichen Öls mit einer Reinheit >98% (ca. 75% d.Th.).

### Beispiel 4: Herstellung von 2-Fluor-2-chlormethyl-malonsäurediethylester (erfindungsgemäß)

100 g des Produkts aus Beispiel 2 wurden bei 0 bis 5°C mit 400 g Thionylchlorid versetzt, wobei sich nach kurzer Zeit in eine klare, braune Lösung bildete. Nach 1 Stunde Nachrühren bei 0 bis 5°C wurden bei dieser Temperatur 5,0 g Triethylamin zugesetzt, die Reaktionsmischung zum Rückfluss aufgeheizt und für 48 Stunden unter Rückfluss erhitzt. Der nach anschließender Einengung bis zur Trockne verbliebene Rückstand wurde in 300 g Dichlormethan aufgenommen und die resultierende Lösung bei Raumtemperatur mit 300 g einer 5 Gew.-%-igen wässrigen Natriumhydrogencarbonat-Lösung neutral gewaschen. Durch Einengen der organischen Phase zur Trockne erhielt man knapp 107 g eines beigen Öls mit einer Reinheit von ca. 91% (ca. 88% d.Th.). Das Produkt konnte zur weiteren Aufreinigung in einer Destillationsausbeute von ca. 80% d.Th. bei 20 mbar fraktioniert destilliert werden.

### Beispiel 5: Herstellung von 2-Fluor-2- chlorsulfinyloxymethyl-malonsäuredimethylester (erfindungsgemäß)

60 g des Produkts aus Beispiel 1 wurden aufgeschmolzen und als Schmelze bei Raumtemp. in vorgelegte 80 g Thionylchlorid eindosiert. Nach 5 Stunden Nachrühren bei Raumtemp. wurde die Reaktionsmischung im Vakuum bis zur Trockne eingeengt. Als Sumpf verblieben 86 g eines beigen Öls mit einer Reinheit von 90% (ca. 90% d.Th.).

### Beispiel 6: Herstellung von 2-Fluor-2-chlormethyl-malonsäuredimethylester aus 2-Fluor-2- chlorsulfinyloxymethyl-malonsäuredimethylester (erfindungsgemäß)

68 g des Produkts aus Beispiel 5 wurden bei 20 mbar fraktioniert destilliert. Man erhielt als Destillat 27,7 g eines farblosen Öls mit einer Reinheit von knapp 97% (ca. 60% d.Th.).

### Beispiel 7: Herstellung von 2-Fluoracrylsäuremethylester aus 2-Fluor-2-chlormethyl-malonsäuredimethylester (erfindungsgemäß)

In eine Mischung aus 100 g N-Methyl-pyrrolidon, 70 g Natriumcarbonat und 5 g 2,6-Di-*tert*.-butyl-4-methylphenol wurden bei 300 mbar und 150°C 50 g des Produkts aus Beispiel 3 in ca. 4 h eindosiert. Man erhielt 27 g einer farblosen Flüssigkeit mit einer Reinheit von 88% (ca. 93% d.Th.). Das Produkt konnte zur weiteren Aufreinigung unter Zusatz von 2,6-Di-*tert*.-butyl-4-methylphenol in einer Destillationsausbeute von ca. 92% d.Th. unter Vakuum fraktioniert destilliert werden.

### Beispiel 8: Herstellung von 2-Fluoracrylsäuremethylester aus 2-Fluor-2-hydroxymethyl-malonsäuredimethylester (erfindungsgemäß)

Eine bei Raumtemp. hergestellte Mischung aus 50 g N-Methyl-pyrrolidon, 30 g Natriumcarbonat, 2,5 g 2,6-Di-*tert*.-butyl-4-methylphenol und 50 g des Produkts aus Beispiel 1 wurde im Vakuum bei 300 mbar langsam auf 150°C aufgeheizt. Das dabei anfallende Destillat wurde bei 0°C mit einer 20 Gew.-%-igen wässrigen Natriumchlorid-Lösung Methanol-frei gewaschen. Man erhielt 24 g einer farblosen Flüssigkeit mit einer Reinheit von 96% (ca. 82% d.Th.).

Das Produkt konnte zur weiteren Aufreinigung unter Zusatz von 2,6-Di-*tert*.-butyl-4-methylphenol in einer Destillationsausbeute von ca. 92% d.Th. unter Vakuum fraktioniert destilliert werden.

### Beispiel 9: Herstellung von 2-Fluoracrylsäuremethylester aus 2-Fluor-2-chlorsulfinyloxymethyl-malonsäuredimethylester (erfindungsgemäß)

In eine Mischung aus 40 g N-Methyl-pyrrolidon, 32 g Natriumcarbonat und 1,4 g 2,6-Di-*tert*.-butyl-4-methylphenol wurden bei 300 mbar und 130°C 48,6 g des Produkts aus Beispiel 5 in ca. 4 h eindosiert. Das dabei anfallende Destillat wurde bei 0°C mit Wasser gewaschen. Man erhielt 14,8 g einer farblosen Flüssigkeit mit einer Reinheit von knapp 88% (ca. 82% d.Th.).

### Beispiel 10: Herstellung von 2-Fluoracrylsäureethylester aus 2-Fluor-2-chlormethyl-malonsäurediethylester (erfindungsgemäß)

In eine Mischung aus 100 g N-Methyl-pyrrolidon, 24 g Natriumcarbonat und 2,5 g 2,6-Di-*tert*.-butyl-4-methylphenol wurden bei 300 mbar und 140°C 50,7 g des destillierten Produkts aus Beispiel 4 in ca. 3 h eindosiert. Man erhielt als Destillat 16,2 g einer farblosen Flüssigkeit mit einer Reinheit von 80% (ca. 47% d.Th.). Das Rohprodukt konnte durch Wäschen mit einer 20 Gew.-%-igen wässrigen Natriumchlorid-Lösung bei 0°C von Methanol befreit werden.

## Patentansprüche

1. Verfahren zur Herstellung von 2-Halogenacrylsäureestern der Formel (IV) 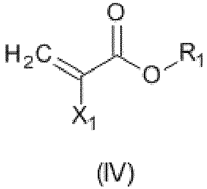 worin
R₁ für C₁-C₆-Alkyl, oder C₃-C₆-Cycloalkyl und
X₁ für Fluor steht,
umfassend den Schritt:
a) Umsetzung von Verbindungen der Formel (II), 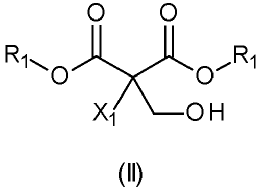 und /oder Verbindungen der Formel (III), 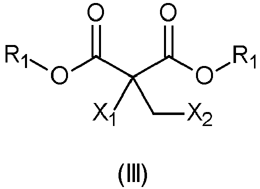
in denen jeweils R₁ und X₁ die für Formel (IV) genannte Bedeutung besitzen und die beiden Reste R₁ identisch oder verschieden, vorzugsweise identisch sind,
und wobei X₂ in Formel (III) für Chlor. Brom oder Chlorsulfinyloxy steht,
in Gegenwart von Base, ausgewählt aus der Gruppe bestehend aus Erdalkali- oder Alkalimetallhydroxide, -amide, -alkoholate, -carbonate, -hydrogenphosphate, -phosphate und in Gegenwart von Lösungsmitteln ausgewählt aus der Gruppe bestehend aus Ethern, Amiden, Sulfonen und Sulfoxiden zu Verbindungen der Formel (IV) bei Temperaturen von 120°C bis 170°C.

2. Verfahren nach Anspruch 1, **dadurch gekennzeichnet, dass** die Verbindungen der Formel (II) durch Hydroxymethylierung von 2-Halogen-malonsäuredialkylestern der Formel (I) mit Formaldehyd hergestellt werden, 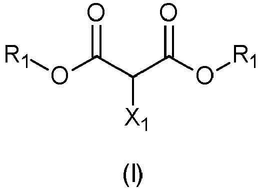 in der R₁ und X₁ die in Anspruch 1 genannten Bedeutungen besitzen.

3. Verfahren nach einem der Anspruch 1 oder 2, **dadurch gekennzeichnet, dass** die Verbindungen der Formel (III) durch Umsetzung von Verbindungen der Formel (II) mit einem Halogenierungsmittel hergestellt werden.

4. Verfahren nach Anspruch 3, **dadurch gekennzeichnet, dass** als Halogenierungsmittel solche eingesetzt werden, die ausgewählt sind aus der Gruppe Thionylchlorid, Thionylbromid, Phosphortrichlorid, Phosphortribromid, Sulfurylchlorid, Sulfurylbromid und Halogenwasserstoffe wie Chlor- oder Bromwasserstoff.

5. Verfahren nach einem der Ansprüche 1 bis 4, **dadurch gekennzeichnet, dass** 2-Fluoracrylsäuremethylester oder 2-Fluoracrylsäureethylester im Schritt a) ausgehend von
• 2-Fluor-2-hydroxymethyl-malonsäuredimethylester bzw. 2-Fluor-2-hydroxymethyl-malonsäurediethylester oder ausgehend von
• 2-Fluor-2-chlormethyl-malonsäuredimethylester bzw. 2-Fluor-2-chlormethylmalonsäurediethylester
hergestellt werden, wobei die Herstellung ausgehend von 2-Fluor-2-chlormethylmalonsäuredimethylester bzw. 2-Fluor-2-chlormethyl-malonsäurediethylester bevorzugt ist.

6. Verfahren nach Anspruch 1, **dadurch gekennzeichnet, dass** 2-Fluoracrylsäuremethylester oder 2-Fluoracrylsäureethylester im Schritt a) ausgehend von
• 2-Fluor-2-chlorsulfinyloxymethyl-malonsäuredimethylester bzw. 2-Fluor-2-chlorsulfinyloxymethyl-malonsäurediethylester
hergestellt werden, wobei die Herstellung ausgehend von 2-Fluor-2-chlorsulfinyloxymethyl-malonsäuredimethylester bzw. 2-Fluor-2-chlorsulfinyl-oxymethylmalonsäurediethylester bevorzugt ist.

7. Verfahren nach einem der Ansprüche 1 bis 6 **dadurch gekennzeichnet, dass** als Basen Alkalimetallhydroxide ausgewählt aus Natriumhydroxid oder Kaliumhydroxid, Alkalimetallamide ausgewählt aus Natriumamid oder Lithiumdiethylamid, Alkalimetallalkoholate ausgewählt aus Natriummethylat oder Kalium-tert-butylat, Alkalimetall-carbonate ausgewählt aus Natriumcarbonat oder Kaliumcarbonat, Alkalimetallhydrogenphosphate ausgewählt aus Dinatriumhydrogenphosphat, Dikaliumhydrogenphosphat, Natriumdihydrogenphosphat oder Kaliumdihydrogenphosphat, oder Alkalimetallphosphate ausgewählt aus Trinatriumphosphat, Trikaliumphosphat eingesetzt werden.

8. Verfahren nach einem der Ansprüche 1 bis 7, **dadurch gekennzeichnet, dass** als Lösungsmittel aus der Gruppe der Ether Dioxan, Amide ausgewählt aus der Gruppe N,N-Dimethyl-formamid, N,N Dimethyl-acetamid und N-Methyl-pyrrolidon, aus der Gruppe Sulfone Sulfolan; und aus der Gruppe Sulfoxide Dimethylsulfoxid eingesetzt werden.

9. Verfahren zur Herstellung von Kunststoffen und polymeren Zusatzstoffen in Arzneimitteln, **dadurch gekennzeichnet, dass** es ein Verfahren gemäß einem der Ansprüche 1 bis 8 umfasst.

10. 2- Chlorsulfinyloxymethyl-2-halogen-malonsäuredialkylester der Formel (III) 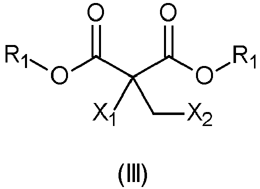 in der
R₁ für C₁-C₆-Alkyl oder C₃-C₆-Cycloalkyl und
X₁ für Fluor steht, und
X₂ für Chlorsulfinyloxy steht.

11. Verbindung der Formel (III) nach Anspruch 10 ausgewählt aus 2-Fluor-2-chlorsulfinyloxymethyl-malonsäuredimethylester und 2-Fluor-2-chlorsulfinyloxymethyl-malonsäurediethylester.

12. Verfahren zur Herstellung von 2-Chlorsulfinyloxymethyl-2-halogen-malonsäuredialkylestern der Formel (III) 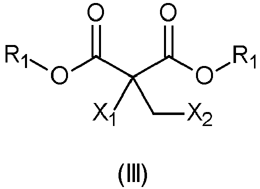 durch Umsetzung von 2-Hydroxymethyl-2-halogen-malonsäuredialkylestern der Formel (II) 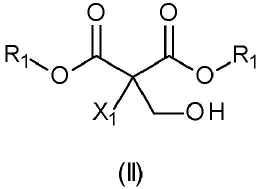 mit Thionylchlorid bei Temperaturen von 0 bis 50°C, wobei in den Formeln (II) und (III) R₁, X₁ und X₂ die im Anspruch 10 angegebene Bedeutung haben.

## Claims

1. Process for preparing 2-haloacrylic esters of the formula (IV) 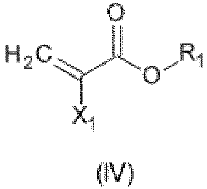 in which
R₁ is C₁-C₆-alkyl or C₃-C₆-cycloalkyl and
X₁ is fluorine,
comprising the step of:
a) reacting compounds of the formula (II) 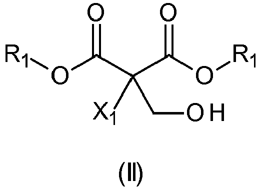 and/or compounds of the formula (III) 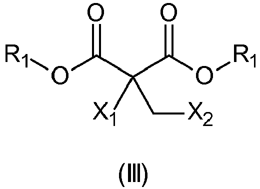
in each of which R₁ and X₁ have the definition given for formula (IV) and the two R₁ radicals are identical or different, preferably identical,
and where X₂ in formula (III) is, chlorine, bromine or chlorosulfinyloxy,
in the presence of base selected from the group consisting of alkaline earth metal or alkali metal hydroxides, amides, alkoxides, carbonates, hydrogenphosphates, phosphates, and in the presence of solvents selected from the group consisting of ethers, amides, sulfones and sulfoxides to give compounds of the formula (IV) at temperatures of 120 C to 170 C.

2. Process according to Claim 1, **characterized in that** the compounds of the formula (II) are prepared by hydroxymethylation of dialkyl 2-halomalonates of the formula (I) with formaldehyde 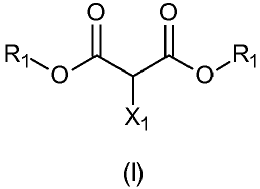 in which R₁ and X₁ have the definitions given in Claim 1.

3. Process according to either of Claims 1 and 2, **characterized in that** the compounds of the formula (III) are prepared by reaction of compounds of the formula (II) with a halogenating agent.

4. Process according to Claim 3, **characterized in that** halogenating agents used are those selected from the group of thionyl chloride, thionyl bromide, phosphorus trichloride, phosphorus tribromide, sulfuryl chloride, sulfuryl bromide and hydrogen halides such as hydrogen chloride and bromide.

5. Process according to any of Claims 1 to 4, **characterized in that** methyl 2-fluoroacrylate or ethyl 2-fluoroacrylate is prepared in step a) proceeding from
• dimethyl 2-fluoro-2-hydroxymethylmalonate or diethyl 2-fluoro-2-hydroxymethylmalonate or proceeding from
• dimethyl 2-fluoro-2-chloromethylmalonate or diethyl 2-fluoro-2-chloromethylmalonate,
preference being given to the preparation proceeding from dimethyl 2-fluoro-2-chloromethylmalonate or diethyl 2-fluoro-2-chloromethylmalonate.

6. Process according to Claim 1, **characterized in that** methyl 2-fluoroacrylate or ethyl 2-fluoroacrylate is prepared in step a) proceeding from
• dimethyl 2-fluoro-2-chlorosulfinyloxymethylmalonate or diethyl 2-fluoro-2-chlorosulfinyloxymethylmalonate,
preference being given to the preparation proceeding from dimethyl 2-fluoro-2-chlorosulfinyloxymethylmalonate or diethyl 2-fluoro-2-chlorosulfinyloxymethylmalonate.

7. Process according to any one of Claims 1 to 6, **characterized in that** bases used are alkali metal hydroxides selected from sodium hydroxide and potassium hydroxide, alkali metal amides selected from sodium amide and lithium diethylamide, alkali metal alkoxides selected from sodium methoxide or potassium tert-butoxide, alkali metal carbonates selected from sodium carbonate and potassium carbonate, alkali metal hydrogenphosphates selected from disodium hydrogenphosphate, dipotassium hydrogenphosphate, sodium dihydrogenphosphate and potassium dihydrogenphosphate or alkali metal phosphates selected from trisodium phosphate, tripotassium phosphate.

8. Process according to any one of Claims 1 to 7, **characterized in that** solvents used are, from the group of ethers, dioxane, amides selected from the group consisting of N,N-dimethylformamide, N,N-dimethylacetamide and N-methylpyrrolidone; from the group of sulfones, sulfolan; and from the group of sulfoxides, dimethylsulfoxide.

9. Process for producing plastics and polymeric additives in medicaments, **characterized in that** it comprises a process according to any of Claims 1 to 8.

10. Dialkyl 2-chlorosulfinyloxymethyl-2-halomalonate of the formula (III) 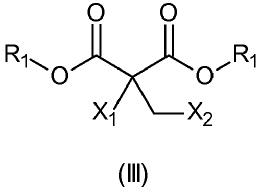 in which
R₁ is C₁-C₆-alkyl or C₃-C₆-cycloalkyl and
X₁ is fluorine, and
X₂ is chlorosulfinyloxy.

11. Compound of the formula (III) according to Claim 10, selected from dimethyl 2-fluoro-2-chlorosulfinyloxymethylmalonate and diethyl 2-fluoro-2-chlorosulfinyloxymethylmalonate.

12. A process for preparing dialkyl 2-chlorosulfinyloxymethyl-2-halomalonates of the formula (III) 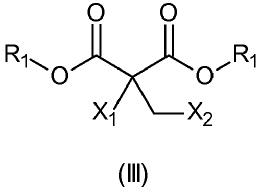 by reacting dialkyl 2-hydroxymethyl-2-halomalonates of the formula (II) 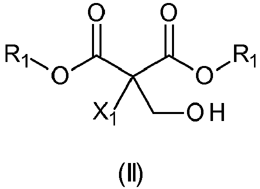 with thionyl chloride at temperatures of 0 C to 50 C, where, in the formulae (II) and (III), R₁, X₁ and X₂ have the definition given in Claim 10.

## Revendications

1. Procédé de fabrication d'esters d'un acide 2-halogénoacrylique de formule (IV) 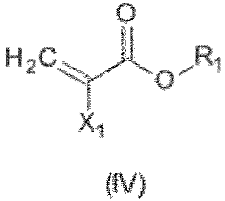 dans laquelle
R₁ représente alkyle en C₁-C₆ ou cycloalkyle en C₃-C₆, et
X₁ représente fluor,
comprenant l'étape suivante :
a) la mise en réaction de composés de formule (II) 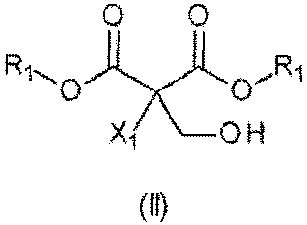 et/ou de composés de formule (III) 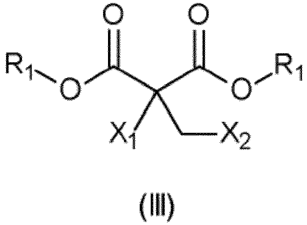
dans lesquelles R₁ et X₁ ont chacun la signification indiquée pour la formule (IV), et les deux radicaux R₁ sont identiques ou différents, de préférence identiques,
et X₂ dans la formule (III) représente chlore, brome ou chlorosulfinyloxy,
en présence d'une base, choisie dans le groupe constitué par les hydroxydes, amides, alcoolates, carbonates, hydrogénophosphates, phosphates de métaux alcalino-terreux ou alcalins, et en présence de solvants choisis dans le groupe constitué par les éthers, les amides, les sulfones et les sulfoxydes pour former des composés de formule (IV) à des températures de 120 °C à 170 °C.

2. Procédé selon la revendication 1, **caractérisé en ce que** les composés de formule (II) sont fabriqués par hydroxyméthylation d'esters dialkyliques d'un acide 2-halogénomalonique de formule (I) avec du formaldéhyde 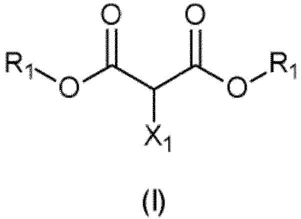 dans laquelle R₁ et X₁ ont les significations indiquées dans la revendication 1.

3. Procédé selon l'une quelconque des revendications 1 ou 2, **caractérisé en ce que** les composés de formule (III) sont fabriqués par mise en réaction de composés de formule (II) avec un agent d'halogénation.

4. Procédé selon la revendication 3, **caractérisé en ce que** des agents d'halogénation choisis dans le groupe constitué par le chlorure de thionyle, le bromure de thionyle, le trichlorure de phosphore, le tribromure de phosphore, le chlorure de sulfuryle, le bromure de sulfuryle et les hydrocarbures halogénés tels que le chlorure ou le bromure d'hydrogène sont utilisés en tant qu'agent d'halogénation.

5. Procédé selon l'une quelconque des revendications 1 à 4, **caractérisé en ce que** l'ester méthylique de l'acide 2-fluoroacrylique ou l'ester éthylique de l'acide 2-fluoroacrylique à l'étape a) est fabriqué à partir de
- l'ester diméthylique de l'acide 2-fluoro-2-hydroxyméthyl-malonique ou l'ester diéthylique de l'acide 2-fluoro-2-hydroxyméthyl-malonique, ou à partir de
- l'ester diméthylique de l'acide 2-fluoro-2-chlorométhyl-malonique ou l'ester diéthylique de l'acide 2-fluoro-2-chlorométhyl-malonique,
la fabrication à partir de l'ester diméthylique de l'acide 2-fluoro-2-chlorométhyl-malonique ou de l'ester diéthylique de l'acide 2-fluoro-2-chlorométhyl-malonique étant préférée.

6. Procédé selon la revendication 1, **caractérisé en ce que** l'ester méthylique de l'acide 2-fluoroacrylique ou l'ester éthylique de l'acide 2-fluoroacrylique à l'étape a) est fabriqué à partir de
- l'ester diméthylique de l'acide 2-fluoro-2-chlorosulfinyloxyméthyl-malonique ou l'ester diéthylique de l'acide 2-fluoro-2-chlorosulfinyloxyméthyl-malonique,
la fabrication à partir de l'ester diméthylique de l'acide 2-fluoro-2-chlorosulfinyloxyméthyl-malonique ou de l'ester diéthylique de l'acide 2-fluoro-2-chlorosulfinyloxyméthyl-malonique étant préférée.

7. Procédé selon l'une quelconque des revendications 1 à 6, **caractérisé en ce qu'**en tant que bases, des hydroxydes de métaux alcalins choisis parmi l'hydroxyde de sodium ou l'hydroxyde de potassium, des amides de métaux alcalins choisis parmi l'amide de sodium ou le diéthylamide de lithium, des alcoolates de métaux alcalins choisis parmi le méthylate de sodium ou le tert-butylate de potassium, des carbonates de métaux alcalins choisis parmi le carbonate de sodium ou le carbonate de potassium, des hydrogénophosphates de métaux alcalins choisis parmi l'hydrogénophosphate de disodium, l'hydrogénophosphate de dipotassium, le dihydrogénophosphate de sodium ou le dihydrogénophosphate de potassium, ou des phosphates de métaux alcalins choisis parmi le phosphate de trisodium, le phosphate de tripotassium sont utilisés.

8. Procédé selon l'une quelconque des revendications 1 à 7, **caractérisé en ce qu'**en tant que solvant du groupe des éthers le dioxane, des amides choisis dans le groupe constitué par le N,N-diméthyl-formamide, le N,N-diméthyl-acétamide et la N-méthyl-pyrrolidone, du groupe des sulfones le sulfolane ; et du groupe des sulfoxydes le diméthylsulfoxyde sont utilisés.

9. Procédé de fabrication de matières plastiques et d'additifs polymères dans des médicaments, **caractérisé en ce qu'**il comprend un procédé selon l'une quelconque des revendications 1 à 8.

10. Esters dialkyliques d'un acide 2-chlorosulfinyloxyméthyl-2-halogéno-malonique de formule (III) 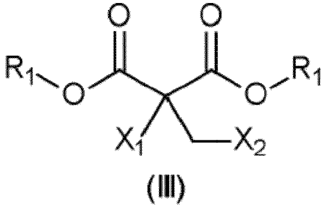 dans laquelle
R₁ représente alkyle en C₁-C₆ ou cycloalkyle en C₃-C₆, et
X₁ représente fluor, et
X₂ représente chlorosulfinyloxy.

11. Composé de formule (III) selon la revendication 10, choisi parmi l'ester diméthylique de l'acide 2-fluoro-2-chlorosulfinyloxyméthyl-malonique et l'ester diéthylique de l'acide 2-fluoro-2-chlorosulfinyloxyméthyl-malonique.

12. Procédé de fabrication d'esters dialkyliques d'un acide 2-chlorosulfinyloxyméthyl-2-halogéno-malonique de formule (III) 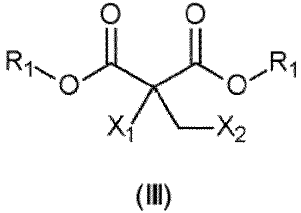 par mise en réaction d'esters dialkyliques d'un acide 2-hydroxyméthyl-2-halogéno-malonique de formule (II) 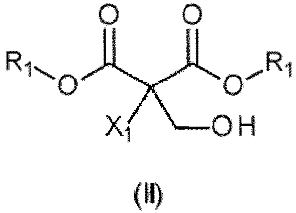 avec du chlorure de thionyle à des températures de 0 à 50 °C, R₁, X₁ et X₂ dans les formules (II) et (III) ayant la signification indiquée dans la revendication 10.
